# EUROPEAN PATENT APPLICATION

(11) **EP 2 641 586 A1**
(43) Date of publication of application: **25.09.2013**
(21) Application number: 11841238.6
(22) Date of filing: 12.10.2011
(51) Int. Cl.: A61K 8/46, A61K 8/73, A61K 8/891, A61K 8/898, A61Q 5/02

(54) **AQUEOUS SHAMPOO**

(30) Priority: 18.11.2010 JP 2010258255
(71) Applicant: Kao Corporation, Chuo-ku Tokyo 103-8210 (JP); Momentive Performance Materials Japan LLC, Tokyo 107-6109 (JP)
(72) Inventor: IWAI, Satoko, Tokyo 131-8501 (JP)
(74) Representative: HOFFMANN EITLE
(86) International application number: PCT/JP2011/005703
(87) International publication number: WO 2012/066722

(57) **Abstract**

The aqueous hair cleansing agent of the present invention comprises the following components (A) to (C):
(A) an anionic surfactant;
(B) side chain amino-modified organo polysiloxane having long chain alkyl at both ends represented by the following general formula (1)

(R²R³₂)SiO-(R³₂SiO)ₓ-(R³R⁴SiO)_{y}-Si(R²R³₂) (1)

wherein formula (1), R² represents linear or branched alkyl group having 12 to 30 carbon atoms 12 to C30, R³ represents substituted or non-substituted monovalent hydrocarbon group having 1 to 6 carbon atoms, and R⁴ represents 3-aminopropyl group or N-(2-aminoethyl)-3-aminopropyl group, and x is an integer number of 1 to 2,000, y is an integer number of 1 to 100, and nitrogen content is 0.31 to 2 mass %;
(C) a cationic galactomannan

and water.

## Description

### [Field of the Invention]

The present invention relates to an aqueous hair cleansing agent.

### [Background of the Invention]

Hair is chemically processed, dried with a hairdryer and treated by other daily hair-care activities to cause damages and fatigue breaking over the hair, which lead to disturbance of hair cuticle, coarse and rough hair. As a result, hair is tangled, and hair is frayed during finger combing or brushing, and forcible combing gives further damage to the hair, which causes frustrated situations for consumers.

For example, Patent Document 1 describes that an aqueous hair cleansing agent containing both of an anionic surfactant and a specific amino-modified silicone provides smoothness with light feeling and a not-heavy manageability feeling to the hair. However, it is also disclosed that an object of providing such smoothness with light feeling is to achieve a smooth combing.

On the other hand, for example, Patent Document 2 discloses a method for receiving and analyzing a signal of vibration generated by combing the hair with a comb or a brush, as a method for evaluating the smoothness of hair.

### [Related Documents]

### [Patent Documents]

[Patent Document 1] Japanese Patent Publication No. JP-A-2008-143859
[Patent Document 2] Japanese Patent Publication No. JP-A-2006-158526

### [Summary of the Invention]

However, the technologies described in the above-described Documents are needed to be improved in the following points. While the combination of the anionic surfactant and the specific amino-modified silicone provides light slipping feel to the hair from the time of being wetted after the time of being dried as described in Patent Document 1, the hair after shampooing and drying with the conventional aqueous hair cleansing agent as described therein is often tangled, and therefore it is necessary to loosen the tangled hair by combing the hair after shampooing and drying with a comb or a brush, and thus there is a room for improvement. Also, while Patent Document 2 describes the method for evaluating the smoothness of the hair, this premises that the hair is combed with a comb or a brush, and this does not include the mention of the unnecessity for combing the hair with a comb or a brush.

The inventor of the present invention has found that a use of an aqueous hair cleansing agent containing a combination of a specific side chain amino-modified organo Polysiloxane having long chain alkyl at both ends and a cationic galactomannan prevents the hair from being frayed and from being tangled both in shampooing and in drying and also provide the hair, which is not easy to be tangled after the drying.

More specifically, the present invention is to provide an aqueous hair cleansing agent containing the following components (A) to (C) :
(A) an anionic surfactant;
(B) side chain amino-modified organo polysiloxane having long chain alkyl at both ends represented by the following general formula (1)

   (R²R³₂) SiO- (R³₂SiO)ₓ- (R³R⁴SiO)_{y}-Si(R²R³₂) (1)

   wherein, in formula (1), R² represents linear or branched alkyl group having 12 to 30 carbon atoms, R³ represents substituted or non-substituted monovalent hydrocarbon group having 1 to 6 carbon atoms, and R⁴ represents 3-aminopropyl group or N-(2-aminoethyl)-3-aminopropyl group, and x is an integer number of 1 to 2,000, y is an integer number of 1 to 100, and nitrogen content is 0.31 to 2 mass %;
(C) a cationic galactomannan
   and water.

Also, the present invention is to provide a use of the above-described aqueous hair cleansing agent for the purpose of being applied to the hair cleansing.

Also, the present invention is to provide a use of the above-described aqueous hair cleansing agent for the purpose of producing a product, which is applied in hair cleansing.

Further, the present invention is to provide a method for cleansing hair by employing the above-described aqueous hair cleansing agent.

According to the present invention, an aqueous hair cleansing agent, which provides reduced tangle of hair after drying and also provides improved fingercomb smoothness, can be achieved.

### DESCRIPTION OF EMBODIMENTS

Aqueous hair cleansing agents of the present invention is described below.

### (A) Anionic Surfactant

Sulfuric acid-based, sulfonic acid-based, and carboxylic cid-based products may typically be employed for component (A) anionic surfactant. For example, the available products include: alkyl sulfate; alkenyl sulfate; polyoxyalkylene alkyl ether sulfate; polyoxyalkylene alkenyl ether sulfate; alkyl sulfosuccinate; polyoxyalkylene alkyl sulfosuccinate; alkanesulfonate; higher fatty acid salts; alkyl ether carboxylate; and the like. Among these, polyoxyalkylene alkyl ether sulfate, polyoxyalkylene alkenyl ether sulfate, alkyl sulfate and alkenyl sulfate are preferable, and further, compounds represented by the following general formula (2) or (3) are preferable.

R¹O(CH₂CH₂O)mSO₃M (2)

R¹OSO₃M (3)

wherein formula (2) or (3), R¹ represents alkyl group or alkenyl group having 10 to 18 carbon atoms, M represents cation derived from alkali metal, alkaline earth metal, ammonium, alkanolamine or basic amino acid, and m represents mass average expressed by number of 1 to 5.

Among these, in view of satisfying both of rapid foaming and improved touch of the foam, it is preferable that R¹ is alkyl group having 12 to 14 carbon atoms and m is the mass average represented by the number of 1 to 2 in the general formula (2), and it is even more preferable to be polyoxyethylene alkyl ether sulfate, in which M is ammonium or sodium.

The component (A) may be employed alone, or a combination of two or more of components thereof, and the content thereof may be, in view of foaming (quantity) and viscosity of the aqueous hair cleansing agent, 1 to 30 mass % in the aqueous hair cleansing agent of the present invention, and more preferably 5 to 25 mass %, and further preferably 8 to 20 mass %, and even more preferably 9 to 15 mass %.

### (B) Side Chain Amino-Modified Organo Polysiloxane Having Long Chain Alkyl At Both Ends

The component (B) is represented by the following general formula (1).

(R²R³₂)SiO-(R³₂SiO)ₓ-(R³R⁴SiO)_{y}-Si(R²R³₂) (1)

wherein formula (1), R² represents linear or branched alkyl group having 12 to 30 carbon atoms, R³ represents substituted or non-substituted monovalent hydrocarbon group having 1 to 6 carbon atoms, and R⁴ represents 3-aminopropyl group or N-(2-aminoethyl)-3-aminopropyl group, and x is an integer number of 1 to 2,000, y is an integer number of 1 to 100, and nitrogen content is 0.31 to 2 mass %.

Here, R² is an alkyl group having 12 to 30 carbon atoms, and may be linear or branched. In view of achieving the aqueous hair cleansing agent, which can simultaneously provides both of enhanced fingercomb smoothness and reduce of tangle in the state of wet hair and enhanced fingercomb smoothness and absence of tangle after drying, alkyl group having 16 to 24 carbon atoms is preferable among these for R², and in view of simultaneously providing absence of tangle during rinsing and after drying, it is more preferable that the component (B) is in the liquid form at 25 degrees C (that is, the viscosity at 25 degrees C is 5 to 100,000 mPa·s). In order to achieve the liquid form at 25 degrees C for the component (B), it is preferable to adopt alkyl group having 16 to 18 carbon atoms when R² is linear, and it is preferable to adopt alkyl group having 16 to 30 carbon atoms when R² is branched, and more preferably branched alkyl group having 16 to 24 carbon atoms is more preferable. Among these, linear alkyl group having 16 to 18 carbon atoms is even more preferable.

R³ is substituted or non-substituted monovalent hydrocarbon group having 1 to 6 carbon atoms, and non-substituted hydrocarbon group typically includes, for example: linear or branched alkyl groups such as methyl group, ethyl group, butyl group, hexyl group and the like; cycloalkyl groups such as cyclohexyl group and the like; alkoxy groups such as methoxy group, ethoxy group, propoxy group, butoxy group and the like; aryl groups such as phenyl group, tolyl group, naphthyl group and the like; aralkyl groups such as benzyl group, β-phenylethyl group, methylbenzyl group and the like; and alkenyl groups such as vinyl group, allyl group and the like. Also, substituted hydrocarbon group typically includes, for example: fluoroalkyl groups such as 3,3,3-trifluoro propyl group and the like. Among these, alkyl groups and aryl groups are preferable, and methyl group and phenyl group are more preferable.

R⁴ is 3-aminopropyl group or N-(2-aminoethyl)-3-aminopropyl group.

In the general formula (1) of the component (B), a plurality of R² may be the same or different, and plurality of R³ may also be the same or different. Further, if a plurality of R⁴ are adopted, they may be the same group or different groups. However, in view of productivity, it is preferable to adopt the same group for R²

The value of x is, in view of obtaining enhanced fingercomb smoothness during rinsing, enhanced smoothness of the hair, and prevent tangle of the hair, an integer number of equal to or higher than 1, and in view of obtaining enhanced finished texture after drying, it is an integer number of equal to or lower than 2,000. Preferably, x is within a range of from 1 to 1,500, and more preferably from 150 to 700, and further preferably within a range of from 200 to 400. The value of y is, in view of obtaining enhanced adsorbability to the hair, an integer number of equal to or higher than 1, and in view of providing non-heavy slipping characteristic of the hair, enhanced fingercomb smoothness and the finished texture without tangle of the hair, it is an integer number of equal to or lower than 100. Preferably, y is within a range of from 1 to 50, and more preferably 2 to 16, and further preferably within a range of from 3 to 7.

Further, in view of obtaining enhanced adsorbability to the hair, the proportion of x and y being y/x is preferably within a specific range of 0.003 to 0.1, which provides the hair with enhanced fingercomb smoothness and without tangle after drying. It is even more preferable to be within a range of 0.004 to 0.05.

In order to provide the above-described specific range of y/x, it is sufficient to adjust the quantities of the raw materials including the quantity of amino group containing silane or amino group containing silicone compound and the quantity of silicone monomer.

The nitrogen content of equal to or higher than 0.31 mass % provides enhanced affinity of amino group of R⁴ in the general formula (1) to the hair so that it is easy to be remained over the hair surface. Consequently, it is preferable that the nitrogen content of component (B) is equal to or higher than 0.31 mass %, in view of firmly exhibiting the conditioning effects such as providing the fingercomb smoothness of the damage hair, the smoothness, and the absence of tangle. Also, in view of preventing the hair from being stickiness in the wet condition or the dried condition and providing enhanced fingercomb smoothness without the tangle of the hair, it is preferable that the nitrogen content of the component (B) is within the range of equal to or lower than 2 mass %. Among the values in such range, the range of from 0.4 to 1.5 mass % is preferable, and 0.4 to 1 mass % is even more preferable. The nitrogen content can be adjusted to be fallen within the desired range by suitably adjusting the quantity of amino group-containing silane or amino group-containing silicone compound.

The nitrogen atom content of the component (B) is calculated by the following procedure. The nitrogen atom content is a content of nitrogen atoms contained in both-end long-chain alkyl and side-chain amino-modified organo polysiloxane and is determined by conducting the neutralization titration.

More specifically, a certain amount of sample (0.5 to 10 g) is dissolved in about 10 folds amount of a mixed solution of isopropyl alcohol and toluene (1:1 [%vol.]). Potassium tetrabromophenolphthalein ethyl ester is added as an indicator, and neutralization titration is carried out using perchloric acid aqueous solution. The content of nitrogen atoms in side chain amino-modified organo polysiloxane having long chain alkyl at both ends is calculated from the amount of sample taken and the equivalent concentration and the consumption of perchloric acid aqueous solution.

Also, the viscosity of the component (B) at 25 degrees C is preferably 5 to 100,000 mPa·s, and more preferably 10 to 50,000 mPa·s. The viscosity of equal to or lower than 100,000 mPa·s can prevent the bad situation, in which the aqueous hair cleansing agent is hard to uniformly spread over the hair surface to cause non-uniform and heavy slipping characteristic and deteriorated fingercomb smoothness, which leads to the state of being tangled. On the other hand, the viscosity of equal to or higher than 5 mPa·s allows the cleansing agent remaining over the hair, exhibiting the effect of actually feeling enhanced fingercomb smoothness without being tangled. Further, viscosity of 50 to 9,500 mPa·s is preferable, and a range of 500 to 4,000 mPa·s is even more preferable.

The viscosity of the component (B) is measured by the following procedure. The viscosity of side chain amino-modified organo polysiloxane having long chain alkyl at both ends compound can be measured by employing a commercially available rotary viscosimeter. Typical commercially available viscometer includes, for example, Brookfield viscometer (Brookfield, United States) and Vismetron viscometer (commercially available from Shibaura System). In this case, Vismetron viscometer (commercially available from Shibaura System) is employed to measure the viscosity of side chain amino-modified organo polysiloxane having long chain alkyl at both ends compound at 25 degrees C.

The conformation of the component (B) when blended into the aqueous hair cleansing agent may be either an oil or emulsion.

The content of the component (B) is preferably equal to or higher than 0.01 mass % in the aqueous hair cleansing agent of the present invention, in view of obtaining improved conditioning effect, and equal to or higher than 0.02 mass % is more preferable, and equal to or higher than 0.03 mass % is even more preferable. Also, the content of the component (B) is preferably equal to or lower than 0.3 mass % in view of providing the finished state without greasiness, and equal to or lower than 0.2 mass % is more preferable, and equal to or lower than 0.1 mass % is even more preferable.

The above-described component (B) can be produced as follows. Side chain amino-modified organo polysiloxane having long chain alkyl at both ends of the component (B) can be produced by an equilibration reaction, which is a general manufacturing process of a silicone compound. More specifically, such method is a method for conducting equilibration of cyclosiloxane such as octamethylcyclotetrasiloxane and the like employed as a silicone monomer with end sequestering agent and amino group-containing silane or amino group-containing silicone compound. This allows producing the component (B).

Alkali catalysts such as sodium hydroxide, potassium hydroxide, cesium hydroxide and the like; quaternary ammonium catalysts such as hydroxylation tetramethylammonium and the like; quaternary phosphonium catalysts such as hydroxylation tetra butyl phosphonium and the like; and all silanolates of the above described compounds can be employed for the catalyst employed in the equilibration reaction in the process for producing the component (B). Also, the adding quantity of the catalyst in the equilibration reaction is within the range of from 0.1 to 1,000 ppm. Preferably, the range is 1 to 500 ppm. The quantity of lower than 0.1 ppm can achieve insufficient progress of the reaction, and on the other hand, the quantity of higher than 1,000 ppm cannot provide sufficient effect that matches the adding quantity.

In the production process for the component (B), any temperature can be employed for the reaction temperature in the equilibration reaction as long as the reaction is progressed at such temperature, and the reaction is ordinarily carried out at a temperature within a range of from 50 to 200 degrees C, and an appropriate reaction temperature is suitably selected according to the employed catalyst. Since siloxane of low molecular weight is created by such equilibration reaction, these siloxane components may be distilled away as required, under the depressurizing condition or atmospheric pressure condition.

### (C) Cationic Galactomannan

The component (C) is a water-soluble cationic polymer, in which quaternary nitrogen-containing group is introduced in a polymer polysaccharide having principal chain containing mannose as a constituting unit is bound with side chain containing galactose as a constituting unit. Galactomannan is obtained from, for example, albumen of seed of legumes. Different physical properties are exhibited according to the relative proportion of mannose and galactose, and: a product containing galactose and mannose at a ratio of 1:1 is fenugreek gum; a product at a ratio of 1:2 is guar gum; a product at a ratio of 1:3 is tara gum; a product at a ratio of 1:4 is locust bean gum; and a product at a ratio of 1:5 is cassia gum. Commercially available products thereof include: Catinal CF -100 (Toho Chemical Industry Co., Ltd.) as cationic fenugreek gum; Jaguar C-13S and Jaguar C-17 (Rhodia) as cationic guar gum; Catinal CT-100 (Toho Chemical Industry Co., Ltd.) as cationic tara gum; Catinal CLB-100 (Toho Chemical Industry Co., Ltd.) as cationic locust bean gum; and Cassia EX-906 (Noveon) as cationic cassia gum. Among these, in view of preventing the hair from being frayed and being tangled during rinsing and after drying and providing enhance fingercomb smoothness, cationic guar gum is more preferable.

The component (C) may be employed alone, or a combination of two or more of components thereof. The content of the component (C) in the aqueous hair cleansing agent may be preferably 0.01 to 3 mass % in the aqueous hair cleansing agent of the present invention, in view of providing suitable foaming, providing smoothness during rinsing and after drying, preventing the hair from being frayed and being tangled during rinsing and after drying, and providing enhanced fingercomb smoothness, and more preferably 0.04 to 1 mass %, and further preferably 0.1 to 0.7 mass %.

In the aqueous hair cleansing agent of the present invention, in view of preventing the hair from being frayed and being tangled during rinsing and after drying and providing enhanced fingercomb smoothness, it is preferable that the mass ratio of the component (C) and the component (B) being (C)/(B) is 1 to 50, and in view of preventing the hair from being frayed and being tangled during rinsing and after drying, 2 to 40 is more preferable, 2 to 20 is even more preferable, and 2 to 14 is all the more preferable.

The aqueous hair cleansing agent of the present invention contains water. It is preferable to employ pure water. The content of water is not particularly limited, and is suitably adjusted according to the applications.

The aqueous hair cleansing agent of the present invention exhibits enhanced fingercomb smoothness and absence of tangle during the rinsing and enhanced fingercomb smoothness and absence of the tangle after drying by employing the combination of the component (B) and the component (C). This avoids the need for combing hair after drying and allows obtaining the hair exhibiting improved finish texture. While the reason for this is not certainly clarified, it is presumed that the component (B) and the component (C) are coacervated during rinsing so that the complex thereof is precipitated on the surface of the hair, and therefore the component (B) can be remained after rinsing. It is further presumed that the component (B) remained on the surface of the hair provides inhibition to the tangle of the hair strands to obtain improved fingercomb smoothness and absence of the tangle. Also, according to the aqueous hair cleansing agent of the present invention, improved foaming characteristic and improved slipping characteristic during shampooing is obtained even if this is applied to the damaged hair.

The advantageous effect of the present invention can be more effectively obtained by employing the aqueous hair cleansing agent of the present invention, which contains, for example, the specific combination of the components (A) to (C) as follows. Such example contain the combination of: polyoxy alkylene alkyl ether sulfate for the component (A); linear both-end long-chain alkyl and side-chain amino-modified organo polysiloxane represented by the general formula (1), in which R² is 16 to 18 carbon atoms for the component (B); and cationic guar gum for the component (C).

The aqueous hair cleansing agent of the present invention may contain other component in addition to the above-described components as required.

### (D) Dimethyl Polysiloxane

The component (D) is dimethyl polysiloxane, and may contain a compound represented by the general formula (4).

R⁵(CH₃)₂SiO-[(CH₃)₂SiO]ₐ-Si(CH₃)₂R⁵ (4)

wherein formula (4), R⁵ represents methyl group or hydroxyl group, and "a" represents the number of 10 to 20,000).

When R⁵ in the general formula (4) is methyl group, the component (D) of "a" of 500 to 10,000 is preferable, and the component (D) of "a" of 1,000 to 5,000 is more preferable.

While various types of dimethyl polysiloxane may be employed, commercially available products include Dow Corning Toray BY11-026, BY22-060 (commercially available from Dow Corning Toray Co., Ltd.), KF-9008, KF-9013, KHE-1 (commercially available from Shin-Etsu Chemical Co., Ltd.) and the like. More specifically, it is represented by the following general formula (5).

(CH₃)₃SiO-[(CH₃)₂SiO]ₐ-Si(CH₃)₃ (5)

wherein formula (5), "a" represents the number of 100 to 20,000.

Dimethyl polysiloxane may be employed in the form of being dissolved or dispersed in a liquid oil (for example, liquid silicone oils such as low-polymerization dimethyl polysiloxane oil, cyclic silicone and the like, or liquid hydrocarbon oils such as isoparaffin and the like).

The component (D) may be preferably in a form of aqueous emulsion. When the aqueous emulsion is prepared, it is preferable to contain at least one type of an emulsifier in view of stabilizing the emulsion.

Typical emulsifier includes, for example: nonionic surfactants such as polyoxyethylene alkyl ether, polyoxyethylene alkylphenylether, polyoxyethylene sorbitan fatty acid ester, polyoxyethylene glyceryl fatty acid ester, polyoxyethylene hydrogenated castor oil, polyethylene glycol fatty acid ester, polyglycerol fatty acid ester saccharose fatty acid ester, poly ether-modified silicone and the like; cationic surfactants such as alkyltrimethylammonium chloride, dialkyl dimethylammonium chloride, alkyl dimethylamine salt, alkoxyalkyl dimethylamine salt, alkylamide alkyl dimethylamine salt and the like; and anionic surfactants such as sodium dodecylbenzenesulfonate, sodium alkylsulfate, ammonium alkylsulfate, sodium polyoxyethylene alkyl ether sulfate, ammonium polyoxyethylene alkyl ether sulfate, potassium cocoate, sodium methyltaurine cocoate and the like.

Typical method for preparing dimethyl polysiloxane emulsion may include a method, in which dimethyl polysiloxane and emulsifying agent are mixed and then water is slowly added while stirring, and mixing at faster speed with a high shearing mixer is carried out at the occasion of the transition from W/O emulsion to O/W emulsion, and then the rest of water is added, and mixing at faster speed with the high shearing mixer is carried out again.

Mean particle diameter of the aqueous emulsion of dimethyl polysiloxane can be arbitrarily changed by the type and quantity of the employed emulsifier and the speed of the stirring rotation of the high shearing mixer. Then, in view of the stability, the smoothness during rinsing and the smoothness after drying, it is preferable to be equal to or smaller than 30 µm. While there is no particular limitation for the lower limit of the particle diameter of the silicone emulsion, it may be, for example, equal to or larger than 0.1 µm in view of the smoothness after drying. Two or more types of these silicone emulsions having different particle diameters may be jointly used.

Further, in view of the stability, the smoothness during rinsing and the smoothness after drying, it is preferable to be present as a dispersed particle of, more preferably equal to or smaller than 10 µm, even more preferably equal to or smaller than 4 µm. In addition to above, the particle diameter of such dimethyl polysiloxane emulsion has the same meaning of the particle diameter of dimethyl polysiloxane in the aqueous hair cleansing agent.

The particle diameter of the dimethyl polysiloxane emulsion is a median diameter measured by a laser light scattering method, and can be determined with a particle size measuring device utilizing laser beam scattering employing, for example, LS-130 commercially available from Beckman Coulter.

The content of the component (D) may be preferably equal to or higher than 0.01 mass % in the aqueous hair cleansing agent of the present invention, in view of obtaining smoothness from the shampooing to the rinsing, and improved fingercomb smoothness and the absence of the tangle after drying, and more preferably equal to or higher than 0.05 mass %, and further preferably equal to or higher than 0.1 mass %. On the other hand, it may be preferably equal to or lower than 5 mass % in view of obtaining appropriate moisture, and more preferably equal to or lower than 3 mass %, further preferably equal to or lower than 2 mass %, and all the more preferably equal to or lower than 0.8 mass %.

In the aqueous hair cleansing agent of the present invention, it is preferable that the mass proportion of the component (D) and the component (B) being (D)/(B) is 1 to 150 in view of preventing the hair from being frayed and being tangled during rinsing and after drying and enhancing the fingercomb smoothness, and from 1 to 100 is preferable, from 1 to 50 is more preferable, and from 1.5 to 20 is even more preferable. Smaller amount of the component (B) may be added to dimethyl polysiloxane of the component (D) to provide enhanced fingercomb smoothness after drying without stickiness.

The aqueous hair cleansing agent of the present invention may be produced by a production process including separately adding the component (B) and the component (D) to a composition containing the component (A) and the component (C) and other components and water, or may be produced by a production process including adding a solution previously prepared by dissolving the component (B) and the component (D).

The aqueous hair cleansing agent of the present invention may contain other component in addition to the above-described components as required.

A nonionic surfactant or an ampholytic surfactant may be added to the aqueous hair cleansing agent of the present invention, in order to further improve the cleansing performance. Also, in order to obtaining enhanced finish texture after drying, a cationic surfactant and/or a cationic polymer may be further blended.

### (Nonionic Surfactant)

The nonionic surfactant typically includes: polyoxyalkylene sorbitan fatty acid esters; polyoxyalkylene sorbitol fatty acid esters; polyoxyalkylene glycerol fatty acid esters; polyoxyalkylene fatty acid esters; polyoxyalkylene alkyl ethers; polyoxyalkylene alkyl phenyl ethers; polyoxyalkylene (hydrogenated) castor oils; saccharose fatty acid esters; polyglycerol alkyl ethers; poly glycerol fatty acid esters; fatty acid alkanolamides; alkyl glycosides; mono alkyl or mono alkenyl glyceryl ethers; and the like.

Among these, polyoxyalkylene sorbitan fatty acid esters such as polyoxyethylene sorbitan fatty acid ester and the like; polyoxyalkylene fatty acid esters such as polyoxyethylene (8 to 20 carbon atoms) fatty acid ester and the like; polyoxyalkylene (hydrogenated) castor oils such as polyoxyethylene hydrogenated castor oil and the like; and alkyl glycoside are preferable.

Also, it is preferable to employ fatty acid alkanolamides, which may be any one of monoalkanol amide, or dialkanol amide, and may also preferably have acyl group having 8 to 18 carbon atoms, and more preferably 10 to 16 carbon atoms. Alternatively, it is preferable to employ that having hydroxyalkyl group having 2 to 3 carbon atoms, and for example, oleic diethanol amide, alkyl alkanolamide (PALM KERNELAMID DEA), coconut diethanolamide, lauric acid diethanolamide, polyoxyethylene coconut monoethanolamide, coconut mono ethanol amide, lauric acid isopropanol amide, lauric acid mono ethanol amide and the like are preferable.

Monoalkyl glyceryl ether or monoalkenyl glyceryl ether are preferable, and concerning alkyl group or alkenyl group contained therein, it is preferable to adopt linear or branched alkyl group having 4 to 10 carbon atoms, and more preferably 8 to 10 carbon atoms. More specifically, such functional group typically includes n-butyl group, isobutyl group, n- pentyl group, 2-methylbutyl group, isopentyl group, n-hexyl group, isohexyl group, n-heptyl group, n-octyl group, 2-ethylhexyl group, n-decyl group, isodecyl group and the like, and 2-ethylhexyl group, isodecyl group and n-octyl group are even more preferable.

### (Ampholytic Surfactant)

Typical ampholytic surfactant may include betaine-based surfactants and the like. Among these, betaine based surfactants such as alkyl dimethylamino acetic acid betaine, fatty acid amide propyl betaine, alkyl hydroxy sulfobetaine and the like are more preferable, and alkyl hydroxy sulfobetaine is even more preferable. Alkyl hydroxy sulfobetaine having acyl group having 8 to 18 carbon atoms is preferable and that 10 to 16 carbon atoms is more preferable, and lauryl hydroxy sulfobetaine and the like is even more preferable.

One of these nonionic or ampholytic surfactants may be employed alone in the aqueous hair cleansing agent, or two or more thereof may be jointly employed, and when the aqueous hair cleansing agent of the present invention is prepared in the form of an aqueous liquid cleansing agent, it is more preferable to employ monoalkyl glyceryl ether or monoalkenyl glyceryl ether and alkyl hydroxy sulfobetaine in addition to the component (A), since this provides enhanced slipping feeling in the foaming and enhanced fingercomb smoothness after drying, as well as providing improved foaming power.

The content of nonionic or ampholytic surfactants may be preferably 0.1 to 10 mass % in the aqueous hair cleansing agent of the present invention, since increased forming effect is obtained. From the above-described point of view, 0.5 to 8 mass % is more preferable, and the range of from 1 to 6 mass % is even more preferable.

### (Cationic Polymer)

Next, the cationic polymers include cationic cellulose, cationic starch, cationic xanthane gum, diallyl quaternary ammonium salt/acrylamide copolymers, vinyl imidazolium trichloride/vinylpyrrolidone copolymers, hydroxyethyl cellulose/dimethyl diallylammonium chloride copolymers, vinylpyrrolidone/quaternized dimethylaminoethyl methacrylate copolymers, polyvinylpyrrolidone/alkylamino acrylate copolymers, polyvinylpyrrolidone/alkylamino acrylate/vinyl caprolactam copolymers, vinylpyrrolidone/methacryl amidopropyl trimethylammonium chloride copolymers alkyl acrylamide/acrylate/alkylamino alkylacrylamide/polyethylene glycol methacrylate copolymers, adipic acid/dimethylamino hydroxypropyl ethylenetriamine copolymer (Cartaretin, commercially available from Sandoz, USA), cationic polymers described in Japanese Patent Publication No. JP-A-S53-139734 (1978) and Japanese Patent Publication No. JP-A-S60-36407 (1985), and the like, and cationic cellulose and diallyl quaternary ammonium salt/acrylamide copolymers are even more preferable.

Also, commercially available products may include, for example: Merquat 550 (ONDEO-NALCO, a copolymer of acrylamide and diallyl dimethyl ammonium salt, CTFA name polyquaternium-7); Luviquat FC370 (BASF, a copolymer of 1-vinyl-2-pyrrolidone and 1-vinyl-3-methylimidazolium salt, CTFA name polyquaternium-16); Gafquat 755N (ISP, a copolymer of 1-vinyl-2- pyrrolidone and dimethylaminoethyl methacrylate, CTFA name polyquaternium-11); Ucare Polymer JR series and Ucare Polymer LR series (Amerchol, salt of a reaction product between trimethyl ammonium substituted epoxide and hydroxyethyl cellulose, CTFA name polyquaternium-10); and Poiz C-60H, Poiz C-80M and Poiz C-150L (Kao Corporation, salt of a reaction product between trimethyl ammonium substituted epoxide and hydroxyethyl cellulose, CTFA name polyquaternium-10), and the like.

Two or more types of these cationic polymers may be jointly employed, and the content thereof may be preferably 0.01 to 3 mass % in the aqueous hair cleansing agent of the present invention, in view of the smoothness from the shampooing to the rinsing, and 0.05 to 2 mass % is more preferable, and 0.1 to 0.5 mass % is even more preferable.

### (Others)

The aqueous hair cleansing agent of the present invention may further contain a pearlescent agent such as ethylene glycol monofatty acid ester, ethylene glycol difatty acid ester, ethyleneglycol monoalkyl ether or ethylene glycol dialkyl ether.

Typical ethylene glycol monofatty acid ester includes ethylene glycol monostearic acid ester, ethylene glycol monobehenic acid ester and the like, and typical ethylene glycol difatty acid ester includes ethylene glycol distearic acid ester, ethylene glycol dibehenic acid ester and the like. Typical ethyleneglycol monoalkyl ether includes ethylene glycol monostearyl ether and the like, and typical ethylene glycol dialkyl ether includes ethylene glycol distearyl ether and the like. Two or more types of these may be jointly employed, and the content thereof may be preferably 0.1 to 5 mass % in the aqueous hair cleansing agent of the present invention, in view of providing improved storage stability of the aqueous hair cleansing agent and improved smoothness during the flaming and the rising, and also providing improved stability of the aqueous hair cleansing agent, and 0.5 to 4 mass % is more preferable, and 1 to 3 mass % is even more preferable.

Also, the aqueous hair cleansing agent of the present invention may contain oil agents as other conditioning agents. Typical oil agents include: hydrocarbons such as squalene, scualane, liquid paraffin, liquid isoparaffin, cycloparaffin and the like; oils and fats such as castor oil, cacao oil, mink oil, avocado oil, olive oil, sunflower oil, camellia oil and the like; waxes such as beeswax, whale wax, lanolin, carnauba wax and the like; higher alcohols such as cetyl alcohol, oleyl alcohol, stearyl alcohol, isostearyl alcohol, 2-octyl dodecanol, glycerol, myristyl alcohol, behenyl alcohol, cetostearyl alcohol and the like; ester oils such as isopropyl palmitate, isopropyl myristate, octyldodecyl myristate, hexyl laurate, cetyl lactate, propyleneglycol monostearate, oleyl oleate, hexadecyl 2-ethylhexanoate, isononyl isononanoate, tridecyl isononanoate and the like; and higher fatty acids such as capric acid, lauric acid, myristic acid, palmitic acid, stearic acid, behenic acid, oleic acid, coconut oil fatty acid, isostearic acid, isopalmitic acid and the like; and additionally, isostearyl glyceryl ether, polyoxypropylene butyl ether and the like. Among these, higher alcohols and oils and fats are preferable, and myristyl alcohol, cetyl alcohol, stearyl alcohol, sunflower oil, and camellia oil are more preferable. One of these oil agents may be employed alone, or two or more thereof may be jointly employed, and the content thereof may be preferably 0.1 to 2 mass % in the aqueous hair cleansing agent of the present invention, and 0.2 to 1.5 mass % is more preferable, and 0.3 to 1.0 mass % is even more preferable.

The aqueous hair cleansing agent of the present invention may contain a viscosity modifier. Typical viscosity modifiers include hydroxyethyl cellulose, methylcellulose, polyethylene glycol, polypropylene glycol, ethylene glycol, propylene glycol, isoprene glycol, ethanol, benzyl alcohol, benzyloxy ethanol, phenoxyethanol, clay mineral, salts (sodium chloride, ammonium chloride, sodium citrate and the like) and the like, and among these, benzyl alcohol, ethanol, polypropylene glycol, sodium chloride, and sodium citrate are preferable. Two or more types of the viscosity modifier may be jointly employed, and the used amount thereof may be preferably 0.01 to 5 mass % in the aqueous hair cleansing agent of the present invention, in view of providing enhanced foam volume and quality of foam, and 0.05 to 4 mass % is more preferable, and 0.1 to 3 mass % is even more preferable.

Other components that are generally employed in the aqueous hair cleansing agent may further be suitably blended in the aqueous hair cleansing agent of the present invention according to the object. Such components typically include, for example, antidandruff agents; vitamin compounds; disinfecting agent; antiinflammatory agents; antiseptic agents; chelate agents; glycylglycine; lanolin acid; moisturizing agents such as glycerol, sorbitol, panthenol and the like; coloring agents such as dyes, pigments and the like; extracted materials such as extracts of eucalyptus in polar solvents , protein obtained from shells having nacreous layer or pearls or hydrolysates thereof, proteins obtained from honey, royal jelly or silk or hydrolysates thereof, protein-containing extracts obtained from seeds of legumes, extracts of panax ginseng, extracts of rice germ, extracts of Fucus, extracts of aloe, extracts of alpinia speciosa leaf, extracts of lotus flower, extracts of licorice, extracts of chlorella and the like; pearlescent agents such as titanium oxide and the like; flavors; coloring agents; UV absorbers; and antioxidants.

The aqueous hair cleansing agent of the present invention may preferably have pH of 2 to 6 (20 fold-diluted with water, at 25 degrees C) at the time of being applied to the hair, in view of providing enhanced gloss and manageability of the hair and improving the anti-tangle of the hair after drying, and pH 3 to 5 is more preferable, and pH 3.5 to 4.5 is even more preferable. Typical pH modifier available here may be organic acids, particularly α-hydroxy acid is preferable, and more specifically, malic acid, citric acid, lactic acid, glycolic acid are preferred. Two or more types of the organic acids may be jointly employed, and the amount thereof may be preferably 0.01 to 5 mass % in the aqueous hair cleansing agent of the present invention, in view of providing enhanced foam quality and enhanced hair flexibility during shampooing, and 0.1 to 3 mass % is more preferable, and 0.3 to 2 mass % is even more preferable. Alternatively, basic compounds such as sodium hydroxide, potassium hydroxide, ammonium chloride and the like may be used as other pH modifiers, in combination with the above-described organic acids.

The aqueous hair cleansing agent of the present invention may be prepared in desired form such as an aqueous solution, an ethanol solution, an emulsion, a suspension, a gel, a liquid crystal, a solid, an aerosol and the like, and a water or lower alcohol may be employed as a solvent, and a form of a liquid product employing water is more preferable. More specifically, the aqueous hair cleansing agent may be applied to, for example, a hair rinse, a hair conditioner, a hair treatment, a hair pack, a hair cream, a conditioning mousse, a hair mousse, a hair spray, a shampoo, a leave-on treatment and the like. The aqueous hair cleansing agent is more preferable to be prepared as the form for the use in washing away, such as shampoo and the like.

The present invention is not limited to the above embodiments, and modifications, improvements and the like are included within the scope of the present invention provided that the features can achieve the object of the present invention. may be contained in the present invention.

### [EXAMPLES]

Table 1 shows details of R², R³, R⁴, x, y, nitrogen contents (mass %) and viscosity (mPa·s) in side chain amino-modified organo polysiloxane having long chain alkyl at both ends (b1) to (b3) represented by the following general formula (1) employed in the following Examples and Comparative Examples. The process for producing these side chain amino-modified organo polysiloxane having long chain alkyl at both ends (b1) to (b3) is as follows.

(R²R³₂)SiO-(R³₂SiO)ₓ-(R³R⁴SiO)_{y}-Si(R²R³₂) (1)

wherein formula (1), R² represents linear or branched alkyl group having 12 to 30 carbon atoms, R³ represents substituted or non-substituted monovalent hydrocarbon group having 1 to 6 carbon atoms, and R⁴ represents 3-aminopropyl group or N-(2-aminoethyl)-3-aminopropyl group, and x is an integer number of 1 to 2,000, and y is an integer number of 1 to 100.

### (Synthesis of Side Chain Amino-Modified Organo Polysiloxane Having Long Chain Alkyl At Both Ends (b1))

First of all, alkyl-modified polyorganosiloxane represented by the following general formula (6) was synthesized.

R^{A}(CH₃)₂-SiO-((CH₃)₂SiO)₂₀-Si(CH₃)₂R^{A} (6)

wherein formula (6), R^{A} represents linear alkyl group having 16 to 18 carbon atoms.

More specifically, 100 parts of a mixture of 1-hexadecene and 1-octadecene (commercially available from Idemitsu Kosan Co., Ltd., linealene 168) and 0.2 part of chloroplatinic acid 1 % isopropanol solution were mixed while stirring and heated to 70 degrees C. Subsequently, 300 parts of both-end hydrogen polyorganosiloxane represented by the following general formula (7) was dropped at 70 to 80 degrees C. After the drop was ended, the solution was stirred at 80 degrees C for 1 hour, and the reaction was completed after it was confirmed that a peak representing Si-H was disappeared in the infrared absorption spectrum.

H(CH₃)₂-SiO-((CH₃)₂SiO)₂₀-Si(CH₃)₂H (7)

Alkyl-modified polyorganosiloxane represented by the general formula (6) exhibiting viscosity of 0.030 Pa·s (measured with No. 1 rotor, 60 rpm, 25 degrees C) was obtained according to this process.

74 parts of alkyl-modified polyorganosiloxane represented by the general formula (6) obtained as described above, 651 parts of octamethylcyclotetrasiloxane and 25 parts of N-(2-aminoethyl)-3-aminopropylmethyl dimethoxy silane were mixed while stirring and heated to 90 degrees C. Subsequently, 90 ppm (represented as an active constituent) of 27 % aqueous solution of hydroxylation tetramethylammonium was added thereto, and an equilibration reaction was conducted at 90 degrees C for 3 hours. Finally, low molecular weight component of siloxane was distilled away under a reduced pressure, to obtain both-end long-chain alkyl and side-chain amino-modified organo polysiloxane (b1) exhibiting nitrogen content of 0.49 mass % and viscosity of 1,200 mPa·s (measured with No. 3 rotor, 30 rpm, 25 degrees C).

100 parts of 2-octyl-1-dodecen represented by the following structural formula (x) and 0.2 part of 1% isopropanol solution of chloroplatinic acid were mixed while stirring and heated to 70 degrees C. Subsequently, 272 parts of both-end hydrogen polyorganosiloxane represented by the general formula (7) was dropped at 70 to 80 degrees C.
After the drop was ended, the solution was stirred at 80 degrees C for 1 hour, and the reaction was completed after it was confirmed that a peak representing Si-H was disappeared in the infrared absorption spectrum.

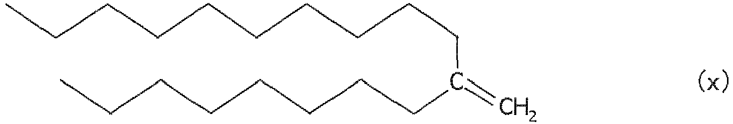

### (Synthesis of Side Chain Amino-Modified Organo Polysiloxane Having Long Chain Alkyl At Both Ends (b2))

A production process, which is similar to the process for producing (b1), was conducted except that 46 part of alkyl-modified polyorganosiloxane represented by the general formula (8), 631 parts of octamethylcyclotetrasiloxane and 74 parts of N-(2-aminoethyl)-3-aminopropylmethyl dimethoxy silane were instead employed, to obtain both-end long-chain alkyl and side-chain amino-modified organo polysiloxane (b2) exhibiting nitrogen content of 0.91 mass % and viscosity of 1,300 mPa·s (measured with No. 3 rotor, 60 rpm, 25 degrees C).

R^{C}(CH₃)₂-SiO-((CH₃)₂SiO)₂₀-Si(CH₃)₂R^{C} (8)

wherein formula (8), R^{C} represents alkyl group represented by the following structural formula (9)

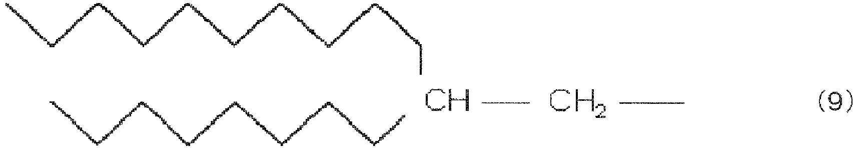

### (Synthesis of Side Chain Amino-Modified Organo Polysiloxane Having Long Chain Alkyl At Both Ends (b3))

A production process, which is similar to the process for producing (b1), was conducted except that 32 parts of alkyl-modified polyorganosiloxane represented by the general formula (6), 707 parts of octamethylcyclotetrasiloxane and 11 parts of N-(2-aminoethyl)-3-aminopropylmethyl dimethoxy silane were instead employed, to obtain both-end long-chain alkyl and side-chain amino-modified organo polysiloxane (b3) exhibiting nitrogen content of 0.22 mass % and viscosity of 19,000 mPa·s (measured with No. 4 rotor, 6 rpm).

**Table 1**

| table 1 | | | | | | | |
|---|---|---|---|---|---|---|---|
| 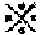 | R² | R³ | R⁴ | x | y | N-CONTENT (%MASS) | VISCOSITY (mPa·s) 25 °C |
| b1 | C₁₆₋₁₈H₃₃₋₃₇ (LINEAR CHAIN TYPE) | CH₃ | N-(2-AMINOETHYL)-3-AMINOPROPYL GROUP | 250∼350 | 3∼4 | 0.49 | 1200 |
| b2 | C₂₀H₄₁ (GUERBET BRANCHED TYPE) | CH₃ | N-(2-AMINOETHYL)-3-AMINOPROPYL GROUP | 250∼350 | 6∼7 | 0.91 | 1300 |
| b3 | C₁₆₋₁₈H₃₃₋₃₇ (LINEAR CHAIN TYPE) | CH₃ | N-(2-AMINOETHYL)-3-AMINOPROPYL GROUP | 600∼700 | 3~4 | 0.22 | 19000 |

### (Examples and Comparative Examples)

Aqueous hair cleansing agents shown in Table 2 was prepared, and was evaluated by the following evaluation method. The results thereof are shown in Table 2. Here, the values of pH were values of 20 fold-diluted with water at 25 degrees C.

### (Evaluation Method)

### (1) Foamability

A 25 cm-long and 5.5 cm-wide human hair bundle of 10 g in weight was lightly rinsed with warm water of 40 degrees C, and then surplus water was wiped off. Then, 0.5 g the aqueous hair cleansing agent was applied and washed for about 30 seconds to sufficiently create the foam. At that occasion, sensory evaluations for the foamability were conducted by utilizing the following 5-step criteria. The evaluations were conducted by five persons, and the integrated values were acquired.
5: very foamable
4: slightly foamable
3: feeling that it is ordinary
2: poorly foamable
1: not foamable.

### (2) Slipping Feeling during Foaming

A 25 cm-long and 5.5 cm-wide human hair bundle of 10 g in weight was lightly rinsed with warm water of 40 degrees C, and then surplus water was wiped off, and 0.5 g the aqueous hair cleansing agent was employed to sufficiently create the foam for about 30 seconds. At that occasion, sensory evaluations for the slipping feeling were conducted by utilizing the following 5-step criteria. The evaluations were conducted by five persons, and the integrated values were acquired.
5: very slippery
4: slightly slippery
3: feeling that it is ordinary
2: poorly slippery
1: not slippery

### (3) Anti-tangle of the Hair during Rinsing

A 25 cm-long and 5.5 cm-wide human hair bundle of 10 g in weight was lightly rinsed with warm water of 40 degrees C, and then surplus water was wiped off, and 0.5 g the aqueous hair cleansing agent was employed to sufficiently create the foam for about 30 seconds. Then, sensory evaluations for the anti-tangle of the hair were conducted by utilizing the following 5-step criteria, while rinsing out the hair bundle having foam adhered thereon with hot water of 40 degrees C of flow velocity of 2 L/min. The evaluations were conducted by five persons, and the integrated values were acquired.
5: hair is not tangled
4: hair is not very tangled
3: feeling that it is ordinary
2: hair is slightly tangled
1: hair is tangled

### (4) Fingercomb Smoothness during Rinsing

A 25 cm-long and 5.5 cm-wide human hair bundle of 10 g in weight was lightly rinsed with warm water of 40 degrees C, and then surplus water was wiped off, and 0.5 g the aqueous hair cleansing agent was employed to sufficiently create the foam for about 30 seconds. Then, sensory evaluations for the fingercomb smoothness of the hair were conducted by utilizing the following 5-step criteria, while rinsing out the hair bundle having foam adhered thereon with hot water of 40 degrees C of flow rate of 2 L/min. The evaluations were conducted by five persons, and the integrated values were acquired.
5: very good fingercomb smoothness
4: slightly good fingercomb smoothness
3: feeling that it is ordinary
2: poor fingercomb smoothness
1: no fingercomb smoothness

### (5) Anti-tangle of the Hair After Drying

The hair was processed under the conditions that are similar to the conditions for the above-described (3), and then, the hair was dried with a dryer, and sensory evaluations of the anti-tangle of the hair after being sufficiently dried were conducted by utilizing the following 5-step criteria. The evaluations were conducted by five persons, and the integrated values were acquired.
5: hair is not tangled
4: hair is not very tangled
3: feeling that it is ordinary
2: hair is slightly tangled
1: hair is tangled

### (6) Fingercomb Smoothness After Drying

The hair was processed under the conditions that are similar to the conditions for the above-described (3), and then, and sensory evaluations of the fingercomb smoothness of the hair after dried were conducted by utilizing the following 5-step criteria. The evaluations were conducted by five persons, and the integrated values were acquired.
5: very good fingercomb smoothness
4: slightly good fingercomb smoothness
3: feeling that it is ordinary
2: poor fingercomb smoothness
1: no fingercomb smoothness

According to Table 2, it was found that the compositions of Examples exhibited enhanced foamability and enhanced slipping feeling in the shampooing, and also exhibited enhanced anti-tangle and enhanced fingercomb smoothness of the hair in the rinsing and enhanced anti-tangle and enhanced fingercomb smoothness of the hair after drying.

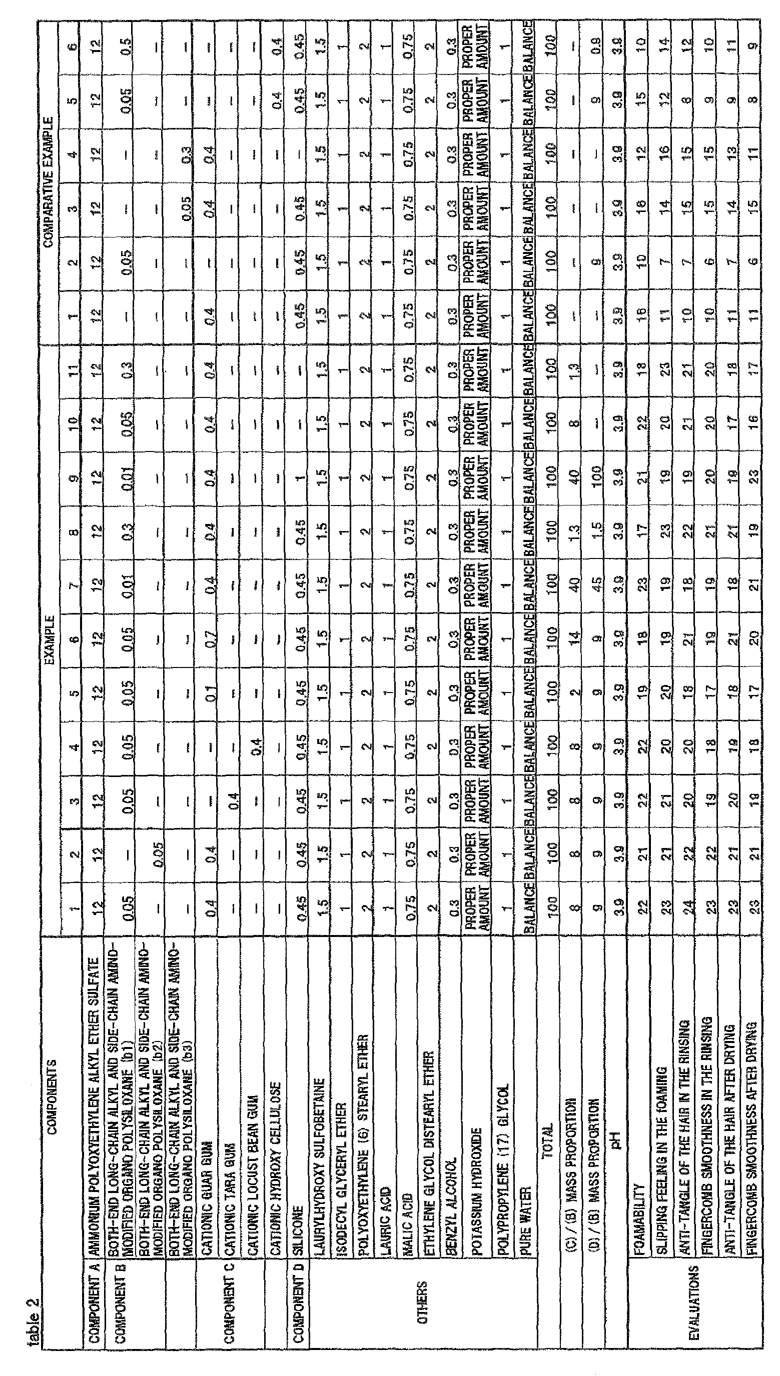

In addition to above, the names and the manufacturer of the raw materials for the respective components in Table 2 are as follows.
* Ammonium polyoxyethylene alkyl ether sulfate
   (name of raw material) Emal 125A (25%)
   (manufacturer) Kao Corporation
* Cationic guar gum
   (name of raw material) Jaguar C-14SK
   (manufacturer) Rhodia
* Cationic tara gum
   (name of raw material) Catinal CT-100
   (manufacturer) Toho Chemical Industry Co., Ltd.
* Cationic locust bean gum
   (name of raw material) Catinal CLB-100
   (manufacturer) Toho Chemical Industry Co., Ltd.
* Cationic hydroxy cellulose
   (name of raw material) Caticelo M-80
   (manufacturer) Kao Corporation
* Silicone (dimethyl polysiloxane)
   (name of raw material) Silicone KHE-1
   (manufacturer) Shin-Etsu Chemical Co., Ltd.
* Laurylhydroxy sulfobetaine
   (name of raw material) Amphitol 20HD (30%)
   (manufacturer) Kao Corporation
* Isodecyl glyceryl ether
   (name of raw material) A product derived from alcohol, in which trimer of propylene is transformed via oxo-process.
* Polyoxyethylene (6) stearyl ether
   (name of raw material) Emulgen 306L
   (manufacturer) Kao Corporation

## Claims

1. An aqueous hair cleansing agent comprising the following components (A) to (C):
(A) an anionic surfactant;
(B) side chain amino-modified organo polysiloxane having long chain alkyl at both ends represented by the following formula (1)
(R²R³₂)SiO-(R³₂SiO)ₓ-(R³R⁴SiO)_{y}-Si(R²R³₂) (1)
wherein formula (1), R² represents linear or branched alkyl group having 12 to 30 carbon atoms, R³ represents substituted or non-substituted monovalent hydrocarbon group having 1 to 6 carbon atoms, and R⁴ represents 3-aminopropyl group or N-(2-aminoethyl)-3-aminopropyl group, and x is an integer number of 1 to 2,000, y is an integer number of 1 to 100, and nitrogen content is 0.31 to 2 mass %);
(C) a cationic galactomannan,
and water.

2. The aqueous hair cleansing agent according to claim 1, wherein the content of the component (B) is 0.01 to 0.3 mass %.

3. The aqueous hair cleansing agent according to claim 1 or 2, wherein the content of the component (C) is 0.01 to 0.3 mass %.

4. The aqueous hair cleansing agent according to any one of claims 1 to 3, wherein the mass ratio of the component (C) and the component (B) being (C)/(B) is 1 to 50.

5. The aqueous hair cleansing agent according to any one of claims 1 to 4, wherein the component (A) is polyoxyalkylene alkylether sulfate, polyoxyalkylene alkenylether sulfate, alkyl sulfate, or alkenyl sulfate.

6. The aqueous hair cleansing agent according to any one of claims 1 to 5, wherein the content of the component (A) is 1 to 30 mass %.

7. The aqueous hair cleansing agent according to any one of claims 1 to 6, wherein x in said formula (1) of the component (B) is an integer number of 200 to 400, and y is an integer number of 3 to 7.

8. The aqueous hair cleansing agent according to any one of claims 1 to 7, wherein the proportion of x and y in said formula (1) of the component (B) being y/x is 0.003 to 0.1.

9. The aqueous hair cleansing agent according to any one of claims 1 to 8, wherein R² in said formula (1) of the component (B) is a linear alkyl group having 16 to 18 carbon atoms.

10. The aqueous hair cleansing agent according to any one of claims 1 to 9, wherein the component (C) is cationic guar gum.

11. The aqueous hair cleansing agent according to any one of claims 1 to 10, further comprising (D) dimethyl polysiloxane.

12. The aqueous hair cleansing agent according to claim 11, wherein the content of the component (D) is 0.01 to 5 mass %.

13. The aqueous hair cleansing agent according to claim 11 or 12, wherein the mass ratio of the component (D) and the component (B) being (D)/(B) is 1 to 150.

14. The aqueous hair cleansing agent according to any one of claims 1 to 13, wherein pH of 20 fold-diluted solution thereof with water at 25 degrees C is 2 to 6.

15. An use of the aqueous hair cleansing agent according to any one of claims 1 to 14 for being applied to hair cleansing.

16. An use of the aqueous hair cleansing agent according to any one of claims 1 to 14 for producing a product, which is applied to hair cleansing.

17. A method for cleansing hair employing the aqueous hair cleansing agent according to any one of claims 1 to 14.
